Europäisches Patentamt

European Patent Office

Office européen des brevets

(19)

(11) Numéro de publication: **0 238 378 B1**

# FASCICULE DE BREVET EUROPEEN

(12)

(45) Date de publication de fascicule du brevet:
**29.01.92**

(51) Int. Cl.⁵: **A61M 5/20**

(21) Numéro de dépôt: **87400324.7**

(22) Date de dépôt: **13.02.87**

(54) **Appareil d'injection ou de prélèvement de substances.**

(30) Priorité: **14.02.86 FR 8602448**

(43) Date de publication de la demande:
**23.09.87 Bulletin 87/39**

(45) Mention de la délivrance du brevet:
**29.01.92 Bulletin 92/05**

(84) Etats contractants désignés:
**AT BE CH DE ES FR GB GR IT LI LU NL SE**

(56) Documents cités:
**FR-A- 2 390 175**
**FR-A- 2 401 667**
**FR-A- 2 466 238**
**FR-A- 2 524 321**
**FR-A- 2 567 760**

(73) Titulaire: **SOCIETE CIVILE DE RECHERCHES MESALYSE**
**4 ter, cours du Général de Gaulle**
**F-21000 Dijon(FR)**

(72) Inventeur: **Charton, Jean-Pierre**
**79 rue Chabot Vharny**
**F-21000 Dijon(FR)**
Inventeur: **Gasquet, Gilbert**
**2 ruelle du Presbytère Rampillon**
**F-77370 Nangis(FR)**

(74) Mandataire: **Nony, Michel**
**Cabinet NONY & CIE, 29, rue Cambacérès**
**F-75008 Paris(FR)**

## Description

L'invention concerne un appareil pour pratiquer des injections ou des prélèvements de substance dans un milieu notamment dans des tissus d'êtres vivants et, plus particulièrement, un appareil pour faire des injections notamment médicamenteuses à usage vétérinaire ou médical et tout spécialement pour la pratique de la mésothérapie humaine, tel que defini dans le preambule de la revendication 1.

Comme on le sait, les injections sont habituellement pratiquées à l'aide d'une aiguille, préalablement ou non associée à une seringue, que l'on plante dans les tissus d'un être vivant.

L'injection peut être intramusculaire ou intraveineuse. Dans certains cas, on souhaite faire une injection en un site particulièrement bien localisé ; ceci nécessite l'implantation de la pointe de l'aiguille en un lieu et à une profondeur bien déterminés. C'est ce qui est recherché en mésothérapie où l'on désire effectuer l'injection directement dans la zône dont la pathologie est justifiable d'un traitement par injection médicamenteuse. Habituellement, l'injection est faite dans le mésoderme c'est-à-dire à une profondeur de l'ordre de quelques dizièmes à plusieurs millimètres de la surface de la peau.

Cette injection peut être faite sous la forme d'un écoulement unique continu ou sous la forme d'un écoulement multiple discontinu en rafale séquencée.

Quel que soit le mode d'administration de la substance médicamenteuse, il est sûr que la mise en place précise de l'extrémité de l'aiguille dans les tissus pose des problèmes, en particulier lorsque celle-ci est plantée à la main car alors seules l'habileté et la dextérité du praticien l'exécutant sont les garants d'une bonne implantation indolore.

Des difficultés de même nature se présentent lorsqu'on procède à des vaccinations ou à des prélèvements quand il faut ponctionner à des profondeurs bien précises.

Un autre secteur d'activité concerné est celui de l'alimentation. Pour allonger la durée d'utilisation ou de conservation des denrées alimentaires il est fréquent de faire des injections d'ozone ou d'antibiotiques par exemple ou de pratiquer des ensemensements.

Pour tenter de remédier aux difficultés liées à l'implantation manuelle des aiguilles dans les tissus vivants on a déjà proposé des appareils pour exécuter plus ou moins automatiquement une telle intervention mais aucun d'eux ne donne entière satisfaction.

En effet, si on utilise un appreil, décrit dans la demande de brevet FR-A-2401667, qui se présente à la manière d'un très gros pistolet pour se substituer à la main de l'opérateur, la mise en oeuvre est délicate; dans un tel cas, l'aiguille associée à une seringue sont propulsées par un mécanisme dans l'épiderme de l'être vivant et la pénétration de l'aiguille est particulièrement douloureuse car la masse animée est très importante du fait de la présence de la seringue pleine, d'où des problèmes de choc et de vibrations non négligeables.

Une autre solution à usage vétérinaire, décrit dans la demande de brevet FR-A-2466238 (sur laquelle se fonde le préambule de la revendication 1) pour de très petits ou de très grands animaux, consiste à désolidariser la seringue de l'aiguille et à réunir ces dernières à l'aide d'une liaison souple pour acheminer la substance médicamenteuse de la seringue à l'aiguille de manière que le praticien n'ait qu'à tenir l'aiguille;l'aiguille est plantée à la main et le fonctionnement de la seringue est quant à lui motorisé. Quel que soit cet avantage, l'implantation de l'aiguille est toujours faite manuellement et si on a recours à des butées de profondeur pour fixer l'importance de la pénétration de l'aiguille dans les tissus, la zone exacte dans laquelle l'implantation est réellement faite est toujours seulement liée à la dextérité du praticien.

Aucune de ces solutions ne convient pour résourdre les diffiultés exposées et, en plus, elles sont particulièrement douloureuses.

Le but de l'invention est de remédier à ces inconvénients.

L'invention a pour objet un appareil pour pratiquer des injections ou des prélèvements de substances notamment médicamenteuses, par exemple dans des tissus d'être vivants ou de denrées alimentaires qui est léger, peu encombrant et très maniable bien qu'entièrement automatique et totalement motorisé de manière à ce que l'implantation de l'aiguille et l'écoulement de la substance à injecter ou à prélever puissent se faire quasi automatiquement et sans douleurs, lorsqu'il s'agit d'êtres vivants, indépendamment de la dextérité du manipulateur.

En outre, du fait de sa conception, l'appareil selon l'invention permet des implanations d'aiguille aussi bien pratiquement normalement au plan superficiel des tissus que des implantations pratiquement tangentiellement à celui-ci et du fait de sa très petite taille relative il convient aussi à des usages stomatologiques ce qu'aucun appareil connu ne permet de faire.

L'invention a pour objet un appareil pour pratiquer des injections ou des prélèvements de substances dans un milieu notamment dans des tissus d'êtres vivants qui comprend une pièce à main destinée à porter une aiguille pour l'implanter dans un milieu, une entité centrale équipée d'un distributeur pour au moins une substance destinée à alimenter l'aiguille, une unité de commande associée entres autres à la pièce à main et au distributeur pour les faire fonctionner et une liaison reliant la pièce à main à l'entité centrale et à l'unité de commande et pour équipée notamment d'une canalisation pour relier l'aiguille au distributeur afin de pouvoir acheminer de la substance du distributeur dans l'aiguille ou inversement.

Cet appareil est notamment caractérisé en ce que la pièce à main comprend un support de petite masse pour recevoir une aiguille amovible et monté mobile en translation entre une position de repos et une position active pour implanter une aiguille et une catapulte pour projeter le support de sa position de repos à sa position active.

L'appareil selon l'invention qui peut être mis en oeuvre dans de nombreux secteurs d'activité trouve une application spécialement en mésothérapie humaine.

D'autres caractéristiques de l'invention apparaîtront à la lecture de la description et des revendications qui suivent et à l'examen du dessin annexé, donné seulement à titre d'exemple, où :

- la Fig.1 est une vue d'un mode de réalisation d'une pièce à main selon l'invention ;
- la Fig.2 est une vue extérieure d'un autre mode de réalisation d'une pièce à main selon l'invention ;
- la Fig.3 est une coupe longitudinale partielle schématique du mode de réalisation de la Fig.2;
- la Fig.3A et 3B sont des coupes suivant les plans A et B respectivement de la Fig.3 ;
- la Fig.3C est une vue perspective partielle d'un mode de réalisation de l'embase d'appui terminale ;
- la Fig.4 est une vue schématique de l'entité centrale et de l'unité de commande ;
- la Fig.5 est une vue de détail schématique de la console de la Fig.4 montrant son compartiment à distributeur où sont placées des seringues ; et
- la Fig.6 est un schéma fonctionnel des connexions.

Pour la commodité de l'exposé, on limitera la description et le dessin à ce qui concerne directement l'invention. Tout ce qui fait appel à des techniques classiques par exemple électriques, hydrauliques, pneumatiques ou automatiques ne sera relaté que sommairement car étant bien connu des spécialistes en la matière.

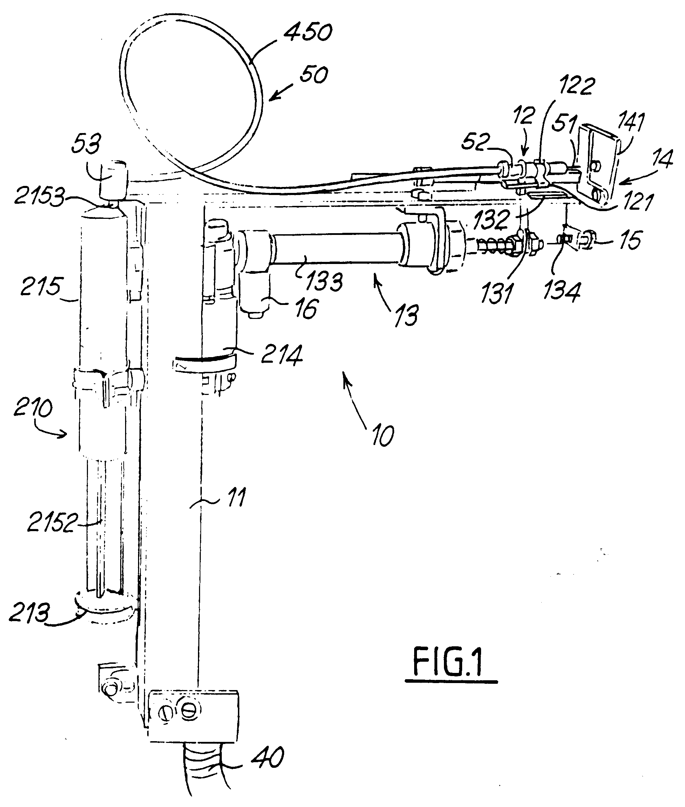

Sur la Fig.1, on a représenté un premier mode de réalisation d'une pièce à main 10 d'un appareil selon l'invention. Comme on le voit, cette pièce à main est dessinée sans ses capots protecteurs servant d'habillage et lui donnant sa présentation finale.

La pièce à main 10 comprend un corps 11 en forme d'équerre sur lequel est monté un support 12 pour recevoir un injecteur 50. Ce support 12 comprend un berceau 121 où reposera un injecteur, comme indiqué par la suite, et une fixation 122 pour maintenir l'injecteur dans son berceau, par exemple des pinces élastiques.

La pièce à main comprend aussi une catapulte 13 avec un chariot 131 auquel est fixé le support 12. Le chariot 131 se déplace en translation sur une voie de guidage 132 faite par exemple de cornières en V ou de glissières classiques par exemple à billes à rattrapage de jeu. De la sorte, le support 12 peut se déplacer avec précision en translation alternative entre une position de repos, effacée, et une position active dans laquelle l'injecteur qui lui est associé est saillant. Le déplacement en translation du chariot 131 est assuré par un propulseur 133 qui projette brusquement le chariot avec son support de sa position de repos, illustrée, à sa position active. Ce propulseur 133 est, par exemple, contitué par un microvérin pneumatique à simple effet à rappel par ressort dont le cylindre est fixé au corps 11 et dont la tige du piston est reliée au support 12 par l'intermédiaire du chariot 131 ; ces fixation et liaison sont assurées par toute technique classique appropriée. A la place du vérin on peut utiliser un électroaimant par exemple agissant par attraction ou répulsion.

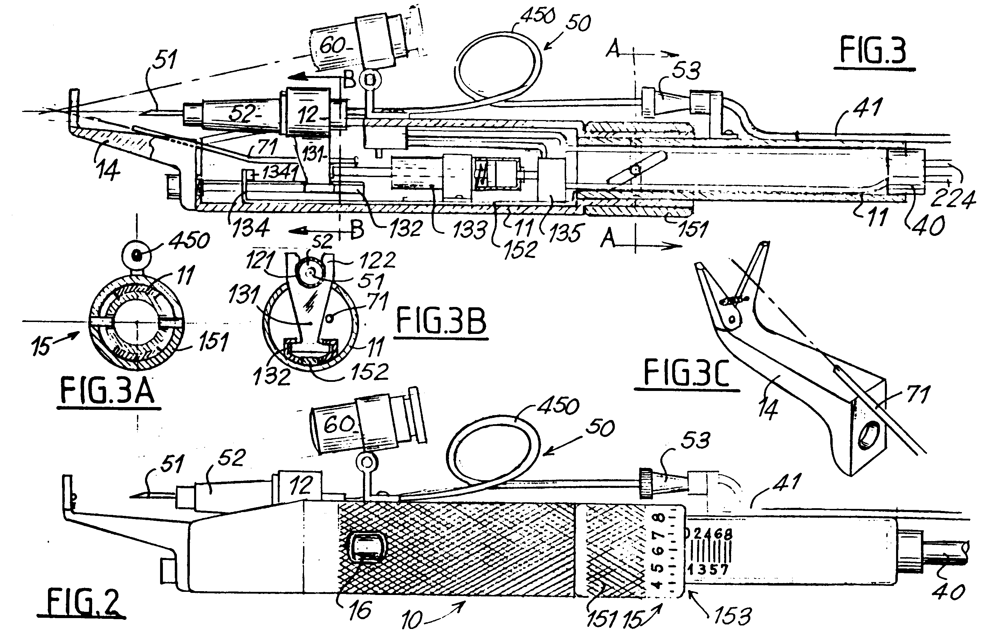

Comme on le voit, la position active du chariot 131 est déterminée par un butoir 134 réglable. La mise en place de ce butoir 134 est commandée par des moyens de réglage 15 par exemple à vis et écrou du type à vernier de palmer ou par tout autre technique analogue. Comme dessiné sur les Fig.3 et 3A, ces moyens de réglage comprennent une molette 151 agissant sur un curseur 152 grâce à une jonction à fente et cheville classique ; une cheville solidaire de la molette traverse à la fois une fente circulaire du corps fixe et une fente hélicoïdale du curseur mobile.

Le butoir est équipé d'un amortisseur 1341 afin que lorsque le chariot est propulsé en position active il s'arrête sans rebond contre le butoir, au besoin avec verrouillage pour l'immobiliser jusqu'à la fin de l'intervention.

Sur l'autre branche du corps 11 est monté un distributeur 210 d'une entité centrale 20. Ce distributeur comprend une seringue 215 classique, maintenue de toute manière appropriée, par exemple un collier à vis, dont le piston 2152 est relié à un poussoir 213 entraîné en translation par un mécanisme à vis et écrou par exemple, à l'aide, par exemple, d'un micromoteur 214 pas à pas reversible dont on peut faire varier le nombre de tours et la vitesse ; on peut ainsi modifier la vitesse de la translation du piston de la seringue et l'amplitude de sa course pour faire sortir de la seringue le volume de substance médicamenteuse souhaité avec la vitesse requise, nécessités par sa nature et son administration.

L'injecteur 50 comprend une aiguille 51 enchassée dans une monture 52, par exemple en matière plastique moulée, et un raccord 53 pour les raisons indiquées par la suite. Au besoin, la monture et le raccord sont reliés par un tronçon de canalisation 450, par exemple un microcatheter.

La monture 52 de l'aiguille 51 est fixée sur le berceau 121 du support 12 par toute technique appropriée par exemple un "circlips" ou une pince. Le raccord 53 est destiné à être connecté à une canalisation 41 mono ou multivoie d'une liaison 40 dont il sera question par la suite et qui, dans ce mode de réalisation, est réduit à son extrême limite.

De la sorte, à chaque patient ou intervention, pour des raisons bien connues d'aseptie et de stérilité, on peut changer facilement et rapidement l'aiguille par remplacement de l'injecteur. Si besoin est, seule l'aiguille 51 enchassée dans sa monture 52 est amovible sans que l'on ait à lui associer un tronçon de canalisation 450 à usage unique.

Un extrémité de la pièce à main 10, voisine du support 12 d'injecteur, est équipée d'une embase d'appui 14, par exemple un pied de biche qui se présente à la manière d'une équerre dont l'une des branches fourchue est divisée en V. Les deux dents de la fourche sont destinées à prendre appui sur le milieu par exemple l'épiderme des tissus d'un patient, de manière à fixer la position relative de l'appareil selon l'invention par rapport à la zone qui présente une pathologie à traiter dans ce cas. La conformation en V du pied de biche permet en outre de tendre les tissus entre ses dents ce qui présente d'autres avantages comme on le verra par la suite. Si nécessaire, pour éviter les inconvénients produits par la formation d'un "coussin" saillant résultant de l'application de l'embase d'appui contre l'épiderme, on articule les deux dents de la fourche à la manière des branches d'un ciseau qui, normalement au repos sont maintenues proches l'une de l'autre. Ces branches voisines s'écartent sous l'action d'un mécanisme approprié, au besoin débrayable à volonté, juste immédiatement avant l'implantation de l'aiguille pour tirer sur les tissus et ainsi aplanir le "coussin" saillant en l'étendant. A la fin de l'intervention, les dents sont rappelées, par exemple par un ressort, à leur position de repos.

Pour augmenter encore la précision d'une implantation d'aiguille résultant de l'utilisation de l'embase d'appui qui sert de guidon de visée, on peut équiper la pièce à main selon l'invention d'une optique grossissante non représentée sur ce mode de réalisation mais illustrée schématiquement sur les Fig.2 et 3. Cette optique 60, par exemple de grossissement x 6, est amovible ou à poste fixe.

On exposera maintenant la manière dont on procède pour se servir de la pièce à main que l'on vient de décrire.

On supposera tout d'abord que cette dernière est reliée à une entité centrale 20 sur laquelle on reviendra par la suite à l'aide de la liaison 40 ; cette entité centrale 20 est destinée à l'alimentation en fluides énergétiques pneumatiques, hydrauliques et électriques ou autres, dont les distributions sont sous la dépendance d'une unité de commande 30, comme indiqué par la suite, qui assure la synchronisation, la coordination et l'enchaînement des phases du processus.

On a garni la seringue 215 en substance médicamenteuse appropriée, par exemple liquide, et on a fixé sur son support 12 une monture 52 avec son aiguille 51, que l'on relie à l'aide du raccord 53 de son tronçon de canalisation 450 à l'embout 2153 de la seringue dont le cylindre 2151 est maintenu sur le corps 11. Le support 12 d'aiguille est en position de repos, c'est-à-dire que l'extrémité effilée ou pointe de l'aiguille est en retrait par rapport à la face du pied de biche de l'embase d'appui 14 qui est en regard d'elle. On agit sur les moyens de réglage 15 (molette 151 des Fig.2 et 3) pour établir la position du butoir 134 afin de fixer la profondeur de la pénétration de l'aiguille qui est indiquée par le curseur 152 qui se déplace devant une graduation 153 (Fig.2 et 3). On choisit la quantité et la vitesse d'injection de la substance à l'aide d'un clavier 311 d'un pupitre 31 de l'unité de commande 30. Le praticien pose le pied de biche de l'embase d'appui 14 contre l'épiderme de la zone à traiter, au besoin en s'aidant de l'optique grossissante de visée, et il appuie sur une détente 16. Ceci actionne l'entité centrale 20 et l'unité de commande 30. S'il y a lieu, le poussoir 213 enfonce un peu le piston de la seringue afin de purger, si nécessaire l'air de la canalisation jusqu'à ce qu'une goutte de substance sourd de la pointe de l'aiguille. Ensuite le vérin du propulseur de la catapulte est actionné, son piston propulsé vers l'avant pour planter l'aiguille dans les tissus du patient qui sont tendus entre les dents en V du pied de biche. Il suffit alors d'attendre la durée choisie pour que l'injection soit faite en continue ou en rafale. Comme on le comprendra par la suite, le bon déroulement des opérations et, éventuellement, la signalisation de tout incident qui pourrait survenir au cours du processus, sont automatiquement rendus perceptibles au praticien grâce à l'unité de commande 30, dont on reparlera par la suite, qui comprend un écran d'affichage 312.

Pour des raisons d'hygiène, on place sur l'embase d'appui un capuchon protecteur amovible 141 qui est changé à chaque patient. Ce capuchon est déformable et extensible sans rupture lorsque l'embase comprend des dents articulées à la manière des branches d'un ciseau.

On se reportera maintenant à la Fig.2, où on a représenté une autre variante de réalisation d'une pièce à main de l'appareil selon l'invention. Les mêmes numéros de référence servent à repérer des constituants homologues.

On remarquera essentiellement que dans ce mode de réalisation la pièce à main ne porte plus le distributeur avec une seringue et son poussoir. La pièce à main se possède plus que le pied de biche de l'embase d'appui, le support d'aiguille et sa catapulte et, éventuellement, l'optique grossissante qui est amovible. La pièce à main est reliée à l'entité centrale 20 par la liaison 40 à circuit multiple sur laquelle on reviendra.

Comme on le remarque sur la Fig.2, du fait du changement de la configuration de la pièce à main, il est possible d'en réduire les dimensions et de l'alléger ; ceci permet en réduisant les masses en mouvement d'éviter ou de bien amoindrir tout problème de rebond et de vibrations qui sont sources de douleurs pour le patient. Dans le mode de réalisation schématisé, la pièce à main ressemble pratiquement à un gros cigare de 300 à 700 g environ ce qui permet, on le comprendra facilement, de l'utiliser même pour des interventions stomatologiques.

Si besoin est, le pied de biche de l'embase d'appui 14 est orientable de manière que les deux branches du V puissent s'incliner et ne pas demeurer pratiquement perpendiculaires à l'axe longitudinal de la pièce à main. En choisissant l'angle d'inclinaison du V du pied de biche, il est possible de modifier l'angle de pénétration de l'aiguille dans les tissus du patient et de pratiquer ainsi des implantations tangentielles.

On ne s'étendra pas plus amplement sur la description de ce mode de réalisation où les mêmes éléments portant les mêmes numéros de référence assurent les mêmes fonctions.

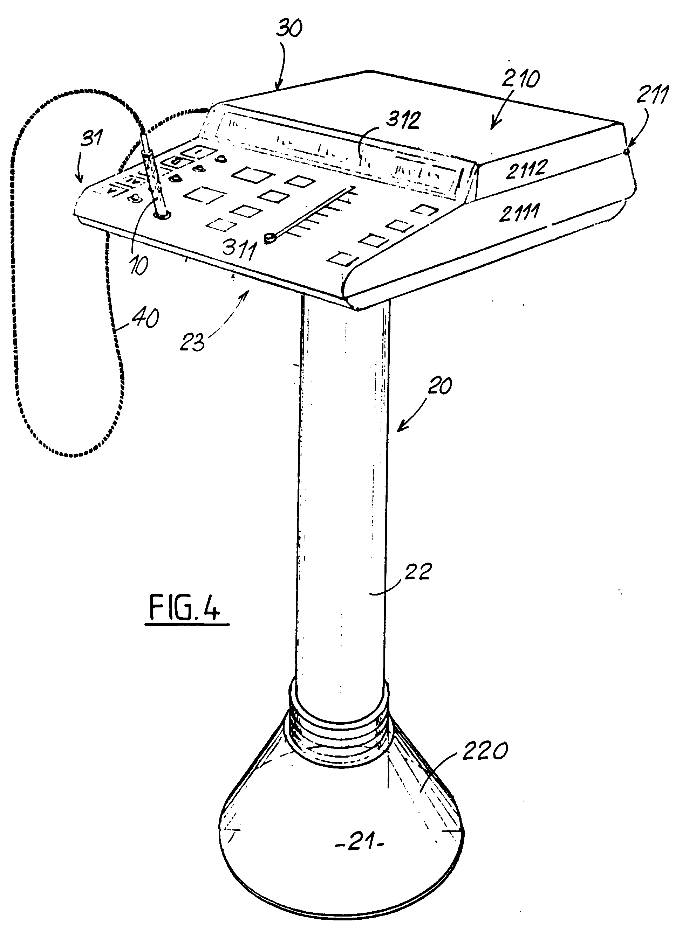

On se reportera maintenant à la Fig.4 où l'on a dessiné sommairement une entité centrale 20. Cette entité se compose essentiellement d'un socle 21, au besoin monté sur roulettes, sur lequel est érigée une colonne 22 qui se termine par une console 23.

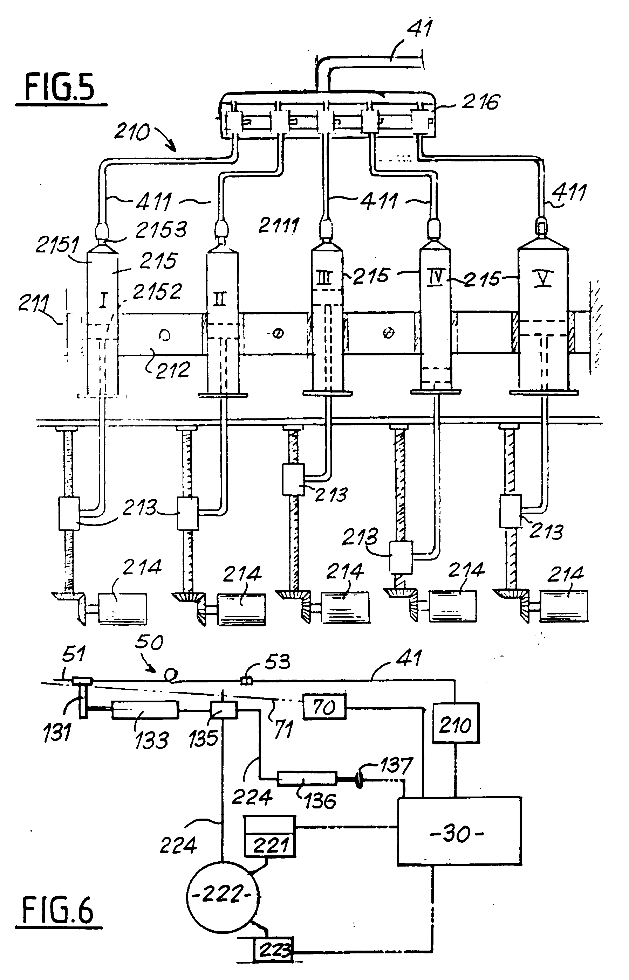

Dans le socle 21 se trouvent logé l'essentiel d'une alimentation 220 en fluides énergétiques, à savoir, par exemple, un compresseur 221 et son réservoir 222 et toutes ses servitudes 223 c'est-à-dire les soupapes de sécurité, les purges, les filtres à poussières et à graisse et à eau, les manostats et le moteur destiné à son entraînement (Fig.6). Tout ceci est classique, on ne s'y étendra donc pas plus amplement. La console 23 qui repose à l'extrémité supérieure de la colonne 22 se présente à la manière d'un pupitre 31 avec un clavier 311 et un écran d'affichage 312.

Le clavier 311 est équipé de touches, de boutons, de curseurs ou analogues, de commande et d'ordres qui permettent de choisir le type d'injection ou de prélèvement, c'est-à-dire des injections continues ou des injections en rafales et, dans ce cas, la séquence, la durée et la fréquence et le nombre des impulsions de chaque rafale. De même, ce clavier permet de choisir la nature de la substance à injecter, la quantité et aussi le debit. On peut aussi télécommander les moyens de réglage 15 et/ou l'inclinaison du pied de biche de l'embase d'appui.

La partie supérieure de la colonne 23 que reçoit le distributeur 210 présente un compartiment 211 qui délimite une chambre 2111 fermée par un couvercle 2112 sous lequel se trouvent un réceptacle 212 destiné à recevoir différentes seringues 215, et des poussoirs 213 animés par un ou des moteurs 214 pour agir sur le piston 2152 de seringue. Le détail de ceci est illustré schématiquement sur la Fig.5. Le cylindre 2151 des seringues est maintenu sur le réceptacle à l'aide de pinces élastiques ou analogue pour rendre leur échange facile

Comme on le sait, pour le traitement de certaines affections il est nécessaire d'avoir recours à l'injection de différentes substances médicamenteuses. Pour ce faire et sans que pour cela il soit nécessaire de pratiquer des implantations successives d'aiguilles différentes, l'appareil est équipé de vanne(s) 216 télécommandée(s) mono et/ou multivoies qui permet(tent) de choisir les substances médicamenteuses à injecter et leur ordre de succession ou de mélange à la suite d'une implantation unique d'une seule aiguille. On chosit les subtances médicamenteuses à injecter à l'aide des touches appropriées du clavier 311.

La coordination et l'enchaînement du bon déroulement de toutes les opérations de fonctionnement peuvent être commodément réglés à l'aide d'un système à microcalculateurs programmables par exemple, de l'unité de commande 30. La manière dont les instructions et les programmes sont établis pour faire fonctionner de tels microcalculateurs sont bien connus des spécialistes et n'entrent pas dans le cadre de l'invention. Comme on le sait, de tels coordination et enchaînement peuvent aussi être assurés par des moyens mécaniques ou électro-mécaniques à l'aide de relais et de moteurs et de cames, comme cela est classique.

Sur l'écran d'affichage 312 apparaîssent, s'il y a lieu, des signaux indiquant les instructions données, les phases en cours du déroulement des opérations, des témoins précisant les organes en service et des signaux d'alarme montrant les incidents ou défaillances.

Sur la Fig.5, on a dessiné schématiquement la manière dont différentes seringues 215 sont montées dans le réceptacle 212 logé dans la chambre 2111 du compartiment 211 du distributeur 210 associé à la console 23. Dans ce mode de réalisation, les diverses conduites particulières 411 débouchent dans la canalisation monovoie 41 par l'entremise de la vanne télécommandée 216. Selon une variante de réalisation,, la canalisation 41 est multivoie et chacune de ses voies correspondant à une seringue ; dans une telle solution, l'extrémité de la canalisation 41 avec ses canaux à laquelle s'adapte le racord 53 se présente à la manière d'un barillet de revolver ou analogue par exemple avec autant d'alvéoles qu'il y a de canaux, la rotation du barillet étant motorisée et si nécessaire télécommandée. Pour cette variante, la ou les vannes 216 peuvent être supprimées. La canalisation 41 multivoie est faite d'un faisceau de microcatheters accolés ou bien d'un seul tenant obtenus par exemple par extrusion simultanée.

Il est clair que l'on peut aussi si nécessaire ajouter en plus des seringues destinées à contenir des substances médicamenteuses, une seringue contenant un produit de rinçage, par exemple du sérum physiologique, lorsqu'il faut nettoyer la canalisation parce que les substances utilisées successivement sont incompatibles ou risquent de réagir et de provoquer des incidents chez le patient et/ou dans l'appareil selon l'invention. Si l'on utilise une canalisation 41 multivoie à plusieurs microcatheters, prévoir un rinçage n'a plus d'intérêt car chaque canal est spécialisé et l'injecteur amovible.

Comme il est classique de le faire, l'appareil est équipé aux endroits appropriés de capteurs de position et/ou de course et de détecteurs de couple ou d'effort entre autres de manière à pouvoir réagir immédiatement à tout incident ou anomalie de fonctionnement qui résulterait d'un blocage d'une seringue, d'une oclusion de la canalisation ou d'un bouchage de l'aiguille elle même, ou de toute fuite sur le circuit. Des signaux correspondants d'alarme apparaîssent alors sur l'écran d'affichage du pupitre, au besoin associés à des signaux acoustiques. Ceci est classique et il n'y a pas lieu de s'y étendre plus longuement.

En se reportant à la Fig.6, on comprend la manière dont les connexions tant pneumatiques qu'électriques et hydrauliques sont assurées.

La liaison 40 comprend la canalisation 41, entre aiguille et distributeur, faite par exemple d'un microcatheter mono ou multivoie ; cette canalisation englobe les conduites 411 entre seringue et canalisation et le tronçon 450 entre raccord 53 et monture 52 d'aiguille. Cette liaison comprend aussi le réseau et conduits 224 reliant le vérin du propulseur 133 au réservoir 222 du compresseur par l'entremise de sa vanne de commande 135 ainsi que ceux reliant la détente 16 à l'unité de commande 30. Le tout est protégé par une gaine appropriée souple, par exemple en matière plastique armée.

Une securité constituée par exemple par un vérin 136 associé à un microinterrupteur, n'autorise le fonctionnement du distributeur 210 que lorsque le support 12 a été effectivement propulsé et l'aiguille 51 plantée dans les tissus.

On observera en particulier que dans le mode de réalisation de la pièce à main des Fig.2 et 3, on obtient une sécurité absolue puisqu'il n'y a aucune liaison ou composant électrique qui y est logé.

Selon une variante d'exécution non illustrée, la détente 16 de la pièce à main commande un microinterrupteur alimenté en très basse tension, selon les normes de sécurité en ce domaine, qui agit sur un ou des relais appropriés.

Si besoin est, on peut placer dans l'entité centrale 20 un laser 70, par exemple He-Ne ou IR, et véhiculer les impulsions lumineuses à l'aide de fibres optiques 71 qui courent dans ou sur la pièce à main. Dans un tel cas, on peut conjuguer par exemple par synergie les effets de la substance médicamenteuse à l'action des impulsions photoniques délivrées par le laser.

Les techniques laser et la manière d'utiliser des fibres optiques pour canaliser un flux lumineux et l'acheminer en un point précis étant bien connues, on ne s'étendra pas davantage sur tout ceci. Ces fibres optiques peuvent aussi servir à conduire le flux lumineux d'un système d'éclairage du champ opératoire, non représenté.

Comme on a pu le constater à la lecture de la description de l'appareil selon l'invention et à la manière de se servir de la pièce à main, grâce à la réduction importante de la masse des pièces en mouvement, il est possible de faire des injections ou des prélèvements précis tant en position qu'en profondeur et cela sans le moindre effet désagréable pour le patient, la dextérité du praticien n'intervenant aucunement.

Si besoin est, pour amoindrir la sensation désagréable qui provoque la pénétration de l'aiguille dans les tissus du patient, on peut munir le support d'appui d'un heurtoir mobile qui est mis en oeuvre de manière à venir frapper les tissus par exemple entre les dents du pied de biche juste avant que l'aiguille propulsée par la catapulte pénètre dans les tissus.

Le choix du déphasage ou décalage temporel entre l'impact du heurtoir et la pénétration de l'aiguille est choisi en fonction des données physiologiques connues concernant les réactions sensitives tactiles et les seuils de leur survenance. Si besoin est, pour tenir compte des caractéristiques physiologiques propres particulières à chaque patient, une pondération peut être prévue pour amplifier ou réduire la durée de ce déphasage. Pour ce faire un bouton approprié est alors placé sur le clavier.

On voit donc tout l'intérêt de l'appareil selon l'invention qui permet de faire des implantations précises tant en position qu'en administration de substances ou prélèvements en s'affranchissant des servitudes liées à la dextérité du manipulateur.

Comme on l'a vu, cet appareil peut convenir aussi bien à des usages médicaux généraux ou spécifiques que vétérinaires de même qu'à des usages alimentaires.

Dans le cas d'une utilisation médicale, l'appareil selon l'invention permet de faire des injections de précisions dans le cuir chevelu, pour des pathologies ORL, de traiter des hydrolipodystrophies localisées, microangiopathiques conjonctives ou sclérosantes, elle permet aussi de traiter les gonarthroses, de pratiquer des injections dans des rides et ridules lors d'intervention de dermoesthétiques, de traiter les digitarthroses et l'utilisation en ondotostomatologie.

Dans ce qui précède on a supposé que les substances par exemple médicamenteuses étaient des fluides liquides mais il est clair qu'en l'absence de contre-indication ou de danger, ces fluides peuvent aussi être gazeux. L'architecture de l'appareil est alors adaptée en conséquence sans qu'il soit nécessaire d'y apporter des bouleversements fondamentaux ; ceci est à la portée du spécialiste en la matière.

L'appareil selon l'invention étant "réversible" en ce qui concerne le sens de fonctionnement du distributeur 210, il est clair que celui-ci peut opérer pour pratiquer des prélèvements et non plus des injections. Les transformations appropriées sont aisées pour le spécialiste.

En utilisant pour l'appareil selon l'invention un microvérin pneumatique pour propulseur de la catapulte on obtient la plantation d'une aiguille dans les tissus avec une très grande vitesse et sans la moindre vibration du fait des très petites masses en mouvement et de la précision du guidage. Grâce à ceci on supprime pratiquement toute douleur et on ne produit pas d'hématome, ce qui favorise une cicatrisation parfaite ; ceci est de la plus haute importance pour les interventions esthétiques sur les rides et ridules.

La profondeur de la pénétration de l'aiguille est ajustée avec précision à l'aide des moyens de réglage qui agissent sur le butoir ; il est clair que ces moyens de réglage peuvent aussi agir sur l'embase d'appui seule ou en relation avec le butoir. De même l'action sur les moyens de réglage peut être motorisée et se faire depuis le pupitre.

Dans les modes de réalisation représentés et décrits on a supposé que la détente déclanchant le fonctionnement était située sur la pièce à main. Il est clair que cette détente peut aussi être logée dans une pédale reliée convenablement à l'entité centrale et à l'unité de commande ; on peut alors opérer au pied.

Pour faire fonctionner l'appareil selon l'invention on utilise un compresseur entraîné par un moteur de 100 W qui permet d'obtenir des pressions atteignant 10 bars alors que seuls six suffisent. Pour alimenter la catapulte on se sert de conduit simple ou multiple fait en matière plastique dénommé "Rilsan" de 6 mm de diamètre environ.

Pour ce qui est de la canalisation, on utilise un ou des microcatheters d'environ 0,6 mm de diamètre intérieur d'environ 2m de longueur. On se sert d'une matière plastique de qualité médicale et, de préférence, le cathéter n'est pas incorporé à la liaison de manière à rester apparent pour, si besoin, pouvoir procéder facilement à son échange.

En ce qui concerne le distributeur on utilise de préférence des seringues de 10cc ou 20cc mais, par exemple pour celle contenant le vluide de rinçage, on peut porter la capacité à 20cc. Pour obtenir le maximum de souplesse pour l'administration des substances médicamenteuses, on se sert de moteurs électriques reversibles pas à pas pour animer les poussoirs. De la sorte, on peut choisir facilement le nombre de goutte à délivrer, leur volume propre et total, la durée de production de chaque goutte et la fréquence de chaque train de gouttes et des différents trains de gouttes. Un seul moteur, avec des transmissions convenables peut aussi animer tous les poussoirs ou bien seulement certains ; s'il y a lieu on associe un moteur particulier à chaque poussoir. Si on utilise un moteur pour chaque poussoir et si chaque conduite particulière à une seringue déterminée est munie s'il y a lieu de sa propre vanne télécommandée, on peut faire facilement des mélanges de substances médicamenteuses. Il est possible d'équiper le distributeur de retours automatiques des poussoirs par exemple en fin de course ou à l'ouverture du couvercle de la chambre du distributeur afin de faciliter l'échange ou le remplacement des seringues. L'utilisation de moteurs électriques facilite grandement l'automatisation par exemple pour ne déclencher l'écoulement d'une substance médicamenteuse seulement qu'après implantation de l'aiguille faite ou bien pour tenir compte des caractéristiques rhéologiques propres aux diverses substances qui par exemple peuvent être mémorisées dans le microcalculateur. Mais il est clair qu'à la place de moteurs électriques on peut aussi se servir de moteurs pneumatiques ou hydrauliques ou même de vérins. Le fait de placer le distributeur ailleurs que dans la pièce à main évite d'agiter les substances qui, dans certains cas, sont particulièrement fragiles et sujettes à cristallisation plus ou moins spontanée.

Enfin on notera que l'entité centrale et l'unité de commande peuvent être aussi logées dans une ou des mallettes pour en faciliter le transport et permettre ainsi des traitements ambulatoires ou à domicile.

Pour faciliter la gestion, l'appareil peut être équipé de compteurs appropriés, associés par exemple à la console, pour décompter le nombre et la nature des interventions ainsi que les quantités de substances utilisées.

**Revendications**

1. Appareil pour pratiquer des injections ou des prélèvements de substance dans un milieu notamment dans des tissus d'êtres vivants comprenant une pièce à main (10) comprenant un support pour recevoir une aiguille amovible mobile en translation, une entité centrale (20) équipée d'un distributeur (210) pour au moins une substance destinée à alimenter l'aiguille et une unité de commande (30) associée au distributeur (210) et à la pièce à main (10) pour les faire fonctionner, une liaison (40) reliant la pièce à main (10) à l'entité centrale (20) et équipée notamment d'une canalisation (41) pour relier l'aiguille au distributeur (210) afin de pouvoir acheminer de la substance du distributeur dans l'aiguille ou inversement, caractérisé en ce que le support (12) de l'aiguille (51) est de faible masse et que la pièce à main (10) comporte une catapulte (13) pour projeter le support (12) de l'aiguille (51) d'une position de repos vers une position active.

2. Appareil conforme à la revendication 1, caractérisé en ce que la catapulte (13) comprend un propulseur (133) fait d'un vérin pneumatique et en ce que l'entité centrale (20) comprend une alimentation en fluides énergétiques (220) et une vanne télécommandée (215) sous la dépendance de l'unité commande (30).

3. Appareil conforme à l'une des revendications 1 et 2, caractérisé en ce que le support (12) est porté par un chariot (131) se déplaçant sur une voie de guidage (132) faite de glissières à rattrapage de jeu.

4. Appareil conforme à l'une quelconque des revendications 1 à 3, caractérisé en ce que la pièce à main (10) comprend une embase d'appui (14) en forme de pied de biche placé sur la trajectoire de l'aiguille et entre laquelle passe cette dernière.

5. Appareil conforme à la revendication 4, caractérisé en ce qu'il comprend des moyens de réglage (15) coopérant avec l'embase d'appui (14) et la position active du support (12) pour déterminer l'importance de la saillie de l'aiguille quand le support de cette dernière est en position active.

6. Appareil selon la revendication 5, caractérisé en ce que ces moyens de réglage (15) agissent sur un butoir (134) fixant la position active du support (12).

7. Appareil conforme à la revendication 5, caractérisé en ce que ces moyens de réglage (15) agissent sur la position de l'embase d'appui (14).

8. Appareil conforme à l'une quelconque des revendications 1 à 7, caractérisé en ce que ce distributeur (210) comprend au moins une seringue (215) à piston (2152) dont le cylindre (2151) est relié à la canalisation (41) par une conduite (411), un poussoir (213) pour agir sur le piston (2152) de la seringue, un moteur (214) pour faire se déplacer ce poussoir et une vanne télécommandée (216) entre seringue et support.

9. Appareil conforme à la revendication 8, caractérisé en ce que ce moteur (214) est à vitesse variable.

10. Appareil conforme à l'une quelconque des revendications 8 et 9, caractérisé en ce que ce moteur (214) est réversible.

11. Appareil conforme à l'une quelconque des revendications 1 à 10, caractérisé en ce que ce distributeur (210) comprend plusieurs seringues (215) à piston (2152) dont les cylindres (2151) respectifs sont tous reliés en parallèle par leur conduite (411) propre à la canalisation (41) et en ce que la vanne télécommandée (215) est une électrovanne à voies multiples ou plusieurs électrovannes sur la conduite (411) entre seringue et support.

12. Appareil selon la revendication 11, caractérisé en ce que la canalisation (41) est multivoie et présente autant de canaux qu'il y a de seringues.

13. Appareil conforme à la revendication 11 ou 12, caractérisé en ce que l'une des seringues (215) contient un fluide de rinçage.

14. Appareil conforme à l'une quelconque des revendications 1 à 13, caractérisé en ce que l'unité de commande (30) est sous la dépendance d'un microcalculateur programmable pour le déroulement du processus.

15. Appareil selon l'une quelconque des revendications 1 à 14, caractérisé en ce qu'il permet de faire une injection unique ou répétitive de séquence réglable.

16. Appareil selon l'une quelconque des revendications 1 à 15, caractérisé en ce que la liaison (40) est equipée d'un conduit (224) pour l'alimentation de la catapulte (13).

17. Appareil conforme à la revendication 16, caractérisé en ce que la liaison (40) ne comprend aucun composant électrique.

18. Appareil conforme à l'une quelconque des revendications 1 à 17, caractérisé en ce qu'il est équipé d'une détente (16) pour provoquer le fonctionnement de l'appareil.

19. Appareil conforme à la revendication 18, caractérisé en ce que la détente (16) est disposée sur la pièce à main (10) pour provoquer un déclenchement manuel.

20. Appareil conforme à la revendication 18, caractérisé en ce que la détente (16) est reliée à l'entité centrale (20) pour provoquer un déclenchement par pédale.

21. Appareil conforme à l'une quelconque des revendications 1 à 20, caractérisé en ce que l'aiguille (51) est solidaire d'une monture (52).

22. Appareil conforme à la revendication 21, caractérisé en ce que la monture (52) est reliée à un tronçon de canalisation (450) avec raccord (53).

23. Appareil conforme à l'une quelconque des revendications 1 à 22, caractérisé en ce que la pièce à main (10) est équipée d'un heurtoir.

24. Appareil conforme à l'une quelconque des revendications 1 à 23, caractérisé en ce que le distributeur (210) comprend une seringue et un poussoir qui sont portés par la pièce à main.

## Claims

1. Apparatus for performing injections or samplings of substances into or from a medium, notably into or from tissues of living beings, the apparatus comprising a hand gun (10) comprising a support for receiving a detachable needle movable in translation, a central assembly (20) equipped with a distributor (210) for at least one substance intended for supplying the needle and a control unit (30) associated with the distributor (210) and with the hand gun (10) for causing them to operate, a connector (40) connecting the hand gun (10) to the central assembly (20) and equipped, notably, with a duct (41) for connecting the needle to the distributor (210) to enable the substance to be fed from the distributor to the needle or inversely, characterized in that the support (12) for the needle (51) is of small mass and in that the hand gun (10) comprises a catapult (13) for projecting the support (12) of the needle (51) from an at-rest position to an active position.

2. Apparatus according to Claim 1, characterized in that the catapult (13) comprises a propulsion device (133) composed of a pneumatic jack and in that the central assembly (20) comprises a supply of energy-carrying fluids (220) and a remotely operated valve (215) under the control of the control unit (30).

3. Apparatus according to one of Claims 1 and 2, characterized in that the support (12) is carried by a carriage (131) moving along a guide track (132) composed of slides designed for taking up play.

4. Apparatus according to any one of Claims 1 to 3, characterized in that the hand gun (10) comprises a cabriole-foot-shaped bearing piece (14) situated on the path of the needle and through which this needle passes.

5. Apparatus according to Claim 4, characterized in that it comprises adjusting means (15) cooperating with the bearing piece (14) and the active position of the support (12) for determining the amount of the projection of the needle when the support for this needle is in the active position.

6. Apparatus according to Claim 5, characterized in that these adjusting means (15) act on a stop (134) fixing the active position of the support (12).

7. Apparatus according to Claim 5, characterized in that these adjusting means (15) act on the position of the bearing piece (14).

8. Apparatus according to any one of Claims 1 to 7, characterized in that this distributor (210) comprises at least one syringe (215) having a piston (2152), of which the cylinder (2151) is connected to the duct (41) by a pipe (411), a pusher (213) for acting on the piston (2152) of the syringe, a motor (214) for causing this pusher to move and a remotely-operated valve (216) between syringe and support.

9. Apparatus according to Claim 8, characterized in that this motor (214) is a variable speed motor.

10. Apparatus according to any one of Claims 8 and 9, characterized in that the motor (214) is reversible.

11. Apparatus according to any one of Claims 1 to 10, characterized in that the distributor (210) comprises a plurality of syringes (215) with pistons (2152), of which the respective cylinders (2151) are all connected in parallel by their own pipes (411) to the duct (41) and in that the remotely operated valve (215) is a multi-way electrically operated valve or a plurality of electrically operated valves on the pipe (411) between syringe and support.

12. Apparatus according to Claim 11, characterized in that the duct (41) is multi-way and has as many channels as there are syringes.

13. Apparatus according to Claim 11 or 12, characterized in that one of the syringes (215) contains a rinsing fluid.

14. Apparatus according to any one of Claims 1 to 13, characterized in that the control unit (30) is subject to a programmable microprocessor for the performance of the process.

15. Apparatus according to any one of Claims 1 to 14, characterized in that it allows a single injection or repetitive injection of adjustable sequence to be performed.

16. Apparatus according to any one of Claims 1 to 15, characterized in that the connector (40) is equipped with a pipe (224) for the supply to the catapult (13).

17. Apparatus according to Claim 16, characterized in that the connector (40) does not comprise any electrical component.

18. Apparatus according to any one of Claims 1 to 17, characterized in that it is equipped with a trigger (16) for causing the apparatus to operate.

19. Apparatus according to Claim 18, characterized in that the trigger (16) is disposed on the hand gun (10) to permit hand starting.

20. Apparatus according to Claim 18, characterized in that the trigger (16) is connected to the central assembly (20) to permit a starting by pedal.

21. Apparatus according to any one of Claims 1 to 20, characterized in that the needle (51) is fixed to a mount (52).

22. Apparatus according to Claim 21, characterized in that the mount (52) is connected to a length of ducting (450) with coupler (53).

23. Apparatus according to any one of Claims 1 to 22, characterized in that the hand gun (10) is equipped with a buffer.

24. Apparatus according to any one of Claims 1 to 23, characterized in that the distributor (210) comprises a syringe and a pusher which are carried by the hand gun.

**Patentansprüche**

1. Vorrichtung zur Durchführung von Injektionen oder Entnahmen von Substanzen in einem Medium, insbesondere in Geweben von Lebewesen, umfassend:

ein Handstück (10) mit einem Träger zur Aufnahme einer abnehmbaren, verschiebbaren Nadel,

eine zentrale Einheit (20) mit einem Verteiler (210) für mindestens eine Substanz, die zum versorgen der Nadel bestimmt ist, und

eine Befehlseinheit (30), die mit dem Verteiler (210) und dem Handstück (10) zu deren Betrieb gekoppelt ist, wobei eine Verbindungseinrichtung (40) das Handstück (10) mit der zentralen Einheit (20) verbindet und zur Verbindung der Nadel mit dem Verteiler (210) insbesondere mit einem Leitungssystem (41) versehen ist, um die Substanz wahlweise vom Verteiler in die Nadel oder umgekehrt leiten zu können,

dadurch gekennzeichnet,

daß der Träger (12) der Nadel (51) eine geringe Masse aufweist, und

daß das Handstück (10) ein Katapult (13) zum Vorschießen des Trägers (12) der Nadel (51) aus einer Ruhelage in Richtung einer aktiven Lage aufweist.

2. Vorrichtung nach Anspruch 1, dadurch gekennzeichnet, daß das Katapult (13) einen von einem Pneumatik-Zylinder gebildeten Antrieb (133) aufweist und daß die zentrale Einheit (20) eine Versorgungseinrichtung für Energie enthaltende Fluide (220) und ein in Abhängigkeit der Befehlseinheit (30) ferngesteuertes Ventil (216) umfaßt.

3. Vorrichtung nach Anspruch 1 oder 2, dadurch gekennzeichnet, daß der Träger (12) von einem Schlitten oder Wagen (131) getragen ist, der sich auf einer Führungsbahn (132) aus nachstellbaren Laufschienen bewegt.

4. Vorrichtung nach einem der Ansprüche 1 bis 3, dadurch gekennzeichnet, daß das Handstück (10) eine UnterstützungsFußfläche (14) in der Form eines Geißfußes umfaßt, die auf der Bahn der Nadel angeordnet ist und durch die die letztere passiert.

5. Vorrichtung nach Anspruch 4, dadurch gekennzeichnet, daß sie eine Regeleinrichtung (15) umfaßt, die mit der Unterstützungs-Fußfläche (14) und der aktiven Position des Trägers (12) zusammenwirkt, um zu bestimmen, wie weit die Nadel hervorragt, wenn sich der Träger der letzteren in der aktiven Position befindet.

6. Vorrichtung nach Anspruch 5, dadurch gekennzeichnet, daß die Regeleinrichtung (15) auf einen die aktive Position des Trägers (12) festlegenden Anschlag (134) wirkt.

7. Vorrichtung nach Anspruch 5, dadurch gekennzeichnet, daß die Regeleinrichtung (15) auf die Lage der UnterstützungsFußfläche (14) wirkt.

8. Vorrichtung nach einem der Ansprüche 1 bis 7, dadurch gekennzeichnet, daß der Verteiler (210) mindestens eine Spritze (215) mit einem Kolben (2152), deren Zylinder (2151) über eine Leitung (411) mit dem Leitungssystem (41) verbunden ist, einen Vorschub (213) zur Wirkung auf den Kolben (2152) der Spritze, einen Motor (214) zur Verschiebung des Vorschubs, und ein ferngesteuertes Ventil (216) zwischen Spritze und Träger umfaßt.

9. Vorrichtung nach Anspruch 8, dadurch gekennzeichnet, daß die Geschwindigkeit des Motors (214) variabel ist.

10. Vorrichtung nach Anspruch 8 oder 9, dadurch gekennzeichnet, daß der Motor (214) umkehrbar ist.

11. Vorrichtung nach einem der Anspruche 1 bis 10, dadurch gekennzeichnet, daß der Verteiler (210) eine Vielzahl von Spritzen (215) mit Kolben (2152) umfaßt, deren entsprechende Zylinder (2151) alle parallel Über ihre eigene Leitung (411) mit dem Leitungssystem (41) verbunden sind, und daß das ferngesteuerte Ventil (216) ein elektrisches Mehrweg-Ventil oder eine Vielzahl von Elektroventilen in der Leitung (411) zwischen Spritze und Träger ist.

12. Vorrichtung nach Anspruch 11, dadurch gekennzeichnet, daß das Leitungssystem (41) mehrwegig ist und so viele Kanäle aufweist wie es Spritzen gibt.

13. Vorrichtung nach Anspruch 11 oder 12, dadurch gekennzeichnet, daß eine der Spritzen (215) eine Spülflüssigkeit enthält.

14. Vorrichtung nach einem der Ansprüche 1 bis 13, dadurch gekennzeichnet, daß die Befehlseinheit (30) zur Steuerung des Prozesses unter der Kontrolle eines programmierbaren Kleinrechners steht.

15. Vorrichtung nach einem der Ansprüche 1 bis 14, dadurch gekennzeichnet, daß sie eine einfache oder in steuerbarer Folge sich wiederholende Injektion erlaubt.

16. Vorrichtung nach einem der Ansprüche 1 bis 15, dadurch gekennzeichnet, daß die Verbindungseinrichtung (40) mit einer Leitung (224) zur Versorgung des Katapults (13) versehen ist.

17. Vorrichtung nach einem dar Ansprüche 1 bis 16, dadurch gekennzeichnet, daß die Verbindungseinrichtung (40) kein elektrisches Teil enthält.

18. Vorrichtung nach einem der Ansprüche 1 bis 17, dadurch gekennzeichnet, daß sie mit einer Auslösung zum Auslösen des Betriebs der Vorrichtung versehen ist.

19. Vorrichtung nach Anspruch 16, dadurch gekennzeichnet, daß die Auslösung (16) am Handstück (10) zum Bewirken einer manuellen Auslösung angeordnet ist.

20. Vorrichtung nach Anspruch 18, dadurch gekennzeichnet, daß die Auslösung (16) mit der zentralen Einheit (20) zum Bewirken einer Pedal-Auslösung verbunden ist.

21. Vorrichtung nach einem der Ansprüche 1 bis 20, dadurch gekennzeichnet, daß die Nadel (51) einstückig mit einem Beschlag (52) ist.

22. Vorrichtung nach Anspruch 21, dadurch gekennzeichnet, daß der Beschlag (52) über ein Teilstück des Leitungssystems (450) mit einem Verbindungsstück (53) verbunden ist.

23. Vorrichtung nach einem der Ansprüche 1 bis 22, dadurch gekennzeichnet, daß das Handstück mit einem Anschlag ausgestattet ist.

24. Vorrichtung nach einem der Ansprüche 1 bis 23, dadurch gekennzeichnet, daß der Verteiler (210) eine Spritze und einen Schieber umfaßt, die vom Handstück gehalten werden.

FIG.1

FIG.3

FIG.3A

FIG.3B

FIG.3C

FIG.2

FIG.4

FIG.5

FIG.6